(19) Europäisches Patentamt
European Patent Office
Office européen des brevets

(11) Veröffentlichungsnummer: **0 163 224 B2**

(12) # NEUE EUROPÄISCHE PATENTSCHRIFT

(45) Veröffentlichungstag der neuen Patentschrift:
**16.10.91 Patentblatt 91/42**

(51) Int. Cl.$^5$: **C07C 303/06**, C07C 303/08

(21) Anmeldenummer: **85106135.8**

(22) Anmeldetag: **18.05.85**

(54) **Verfahren zur Herstellung von Acyloxybenzolsulfonsäuren.**

(30) Priorität: **26.05.84 DE 3419793**

(43) Veröffentlichungstag der Anmeldung:
**04.12.85 Patentblatt 85/49**

(45) Bekanntmachung des Hinweises auf die Patenterteilung:
**21.01.87 Patentblatt 87/04**

(45) Bekanntmachung des Hinweises auf die Entscheidung über den Einspruch:
**16.10.91 Patentblatt 91/42**

(84) Benannte Vertragsstaaten:
**AT BE CH DE FR GB IT LI NL SE**

(56) Entgegenhaltungen:
**DE-A- 2 123 590**
**DE-C- 638 072**
**GB-A- 1 563 994**
**US-A- 2 854 476**
**US-A- 3 503 888**
**INTERSCIENCE PUBLISHERS, 1965, New York, USA; E.E. GILBERT "Sulfonation and Related Reactions", Seiten 10,14,15**
**HOUBEN-WEYL "Methoden der organischen Chemie" 4. Auflage, Band IX, 1955, GEORG THIEME VERLAG, Stuttgart Seiten 503-508, 471, 499 und 503**

(56) Entgegenhaltungen:
**Everett E. Gilbert, Sulfonation and Related Reactions, Interscience Publishers, N. Y., 1965, S. 7-20**
**Chem. Rev., Bd. 62 (1962), S. 574, Abs. 5(a) und (b)**
**Houben-Weyl, "Methoden der Organischen Chemie", Bd. VIII, 1952, S. 543**

(73) Patentinhaber: **BASF Aktiengesellschaft**
**Carl-Bosch-Strasse 38**
**W-6700 Ludwigshafen (DE)**

(72) Erfinder: **Balzer, Wolf-Dieter, Dr.**
**Bruesseler Ring 34**
**W-6700 Ludwigshafen (DE)**
Erfinder: **Bechtolsheimer, Hans-Heinrich, Dr.**
**Ringstrasse 7**
**W-6521 Dittelsheim-Hessloch (DE)**
Erfinder: **Beyer, Karl-Heinz, Dr.**
**Knietschstrasse 6**
**W-6710 Frankenthal (DE)**
Erfinder: **Fikentscher, Rolf, Dr.**
**Von-Stephan-Strasse 27**
**W-6700 Ludwigshafen (DE)**
Erfinder: **Perner, Johannes, Dr.**
**Ginsterweg 4**
**W-6730 Neustadt (DE)**
Erfinder: **Widder, Rudi, Dr.**
**in der Taesch 7**
**W-6906 Leimen (DE)**
Erfinder: **Wolf, Helmut**
**Im Zollstock 6**
**W-6733 Hassloch (DE)**

EP 0 163 224 B2

## Beschreibung

Die Erfindung betrifft ein Verfahren zur Herstellung der Alkali- und Erdalkalisalze von Acyloxybenzosulfonsäuren durch Sulfonierung von Phenol in Gegenwart einer geringen Mengen eines Komplexbildners für das sulfonierende Agens $SO_3$ oder Chlorsulfonsäure, anschließende Veresterung und Neutralisation zum Alkali- oder Erdalkalisalz.

Es ist bekannt, daß Acyloxybenzolsulfonsäuren als aktivierte Ester Acylierungsmittel für Amine, Mercaptane, Wasserstoffperoxid und andere Verbindungen mit aktivem Wasserstoff sind. Für manche Anwendungen, wie die Acylierung von Feststoffen oder von wasserunlöslichen polymeren Verbindungen oder bei der Anwendung in Waschmitteln als Kaltbleichaktivatoren, beispielsweise gemäß EP-Anmeldung 28432, GB-PS 864 798, US-PS 4 412 934 oder der DE-AS 2 602 510, sind wasserlösliche Acylierungsmittel, wie die Salze von Acyloxybenzolsulfonsäuren, z. B. die bekannten Benzoyl- oder Acetyl-p-oxybenzolsulfonate, zweckmäßig.

Weiterhin geht beispielsweise aus der US-PS 3 503 888 die Verwendung von Salzen von Acyloxybenzolsulfonsäuren in Toilettenseifen hervor. In der US-PS 3 503 888 wird eine Verfahrensweise zur Herstellung von Acyloxybenzosulfonsäuren beschrieben, bei der man Phenol mit $SO_3$ sulfoniert und die erhaltene Phenolsulfonsäure mit einem Fettsäurechlorid verestert.

Wesentlich für die Herstellung von Acyloxybenzolsulfonsäuren ist, daß wegen ihrer Hydrolyseempfindlichkeit unter Ausschluß von Wasser gearbeitet werden muß. Aus diesem Grunde scheidet Schwefelsäure als übliches Sulfonierungsmittel für Phenol aus. Nachteilig an dem in der US-PS 3 503 888 beschriebenen Verfahren ist, daß die Sulfonierung nicht optimal abläuft. Sie verläuft unter Bildung von Gemischen aus o- und p-Isomeren und es entsteht eine Reihe unerwünschter Nebenprodukte, wie Sulfone und deren Folgeprodukte, die eine spätere Aufarbeitung im Hinblick auf ein gut rieselfähiges Salz stören können, da sie eine leichte Verbackbarkeit verursachen.

Der Monographie E. E. Gilbert, Sulfonation and Related Reactions, Interscience Publishers John Wiley a. Sons, New York, 1965, Kapitel 1, kann beispielsweise entnommen werden, daß $SO_3$ und Chlorsulfonsäure mit den verschiedensten organischen Verbindungen, wie Aminen, Pyridin, Ethern, Amiden u. a., Komplexe (meist 1 : 1-Addukte) bilden und daß diese Komplexe mildere Sulfonierungsreagentien sind als $SO_3$ oder Chlorsulfonsäure selbst. Durch diese Komplexbildung kann die Reaktivität des sulfonierenden Reagens beeinflußt werden. In der Regel laufen die Sulfonierungen mit diesen Komplexen bei höheren Temperaturen ab als ohne Komplexbildner und werden häufig in Gegenwart eines inerten Lösungsmittels oder eines Überschusses an Komplexbildner durchgeführt. Die Durchsicht der genannten Literaturstelle zeigt, daß die Reaktivität eines solchen Komplexes im Hinblick auf das zu sulfonierende Substrat nicht ohne weiteres vorausgesagt werden kann.

Aufgabe der Erfindung ist es, ein in technischem Maßstab leicht durchzuführendes Verfahren zur Herstellung der Alkali- und Erdalkalisalze von Acyloxybenzolsulfonsäuren zur Verfügung zu stellen, durch das die Salze in hoher Reinheit und in guten Ausbeuten zugänglich werden.

Es wurde nun überraschenderweise gefunden, daß die Sulfonierung von Phenol vorteilhaft bei niederen Temperaturen in Gegenwart von wesentlich geringeren Mengen an Komplexbildnern als es einem 1 : 1-Komplex entspricht, durchgeführt werden kann.

Gegenstand der Erfindung ist ein Verfahren zur Herstellung der Alkali- und Erdalkalisalze von Acyloxybenzolsulfonsäuren der Formel I

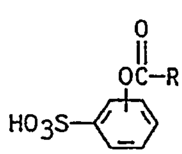

in der R einen geradkettigen oder verzweigten gesättigten Alkylrest mit 5 bis 11 C-Atomen oder einen Phenylrest bedeutet, durch Sulfonierung von Phenol mit $SO_3$ oder Chlorsulfonsäure, anschließende Veresterung und Salzbildung dadurch gekennzeichnet, daß man die Sulfonierung in Gegenwart von 0,2 bis 20 Mol% eines Komplexbildners für $SO_3$ oder Chlorsulfonsäure, bezogen auf das $SO_3$ oder die Chlorsulfonsäure, bei Temperaturen von 20 bis 80°C durchführt wobei man den Komplexbildner dem geschmolzenen Phenol zusetzt und anschließend das Sulfonierungsmittel zusetzt und die erhaltene Phenolsulfonsäure direkt mit einem Säurechlorid der Formel Cl-COR, in der R die für die Formel I genannten Bedeutungen hat, bei 25 bis 55°C umsetzt und anschließend zur Neutralisation die erhaltene flüssige Acyloxibenzolsulfonsäure der Formel I mit einer wäßrigen Lösung von Alkali- oder Erdalkali in Form des Hydroxids, Carbonats oder Bicarbonats in Wasser bei Temperaturen von 0 bis 60°C unter guter Durchmischung so zusammenbringt, daß ein pH-Wertbereich von 3,0 bis 5,5 eingehalten

wird, und man gegebenenfalls das erhaltene Salz in an sich üblicher Weise aus der wäßrigen Lösung isoliert.

Das Wesentliche der Erfindung besteht in dem Zusatz von geringen Mengen eines für $SO_3$ und Chlorsulfonsäure komplexbildend wirkenden Agens, wie es beispielsweise der gennanten Monographie von Gilbert entnommen werden kann. Dieser Zusatz gestattet, die Sulfonierung bei relativ niederen Temperaturen mit guten Ausbeuten und hoher Reinheit an Phenolsulfonsäure durchzuführen. Erstaunlicherweise reagieren die bekannten 1 : 1-Addukte des $SO_3$ oder der Chlorsulfonsäure in dem erfindungsgemäßen Temperaturbereich nicht oder nur sehr langsam. Zudem bereitet eine größere Menge des eingesetzten Komplexbildners, z. B. eines Amins, zusätzlichen Aufwand bei der Aufarbeitung, wenn ein möglichst reines Produkt erhalten werden soll.

In einzelnen seien als komplexbildend wirkende Verbindungen genannt Dioxan, Polyalkylenoxide, wie Diethylen- und Dipropylenglykol, deren Endgruppen durch Alkylreste mit 1 bis 18 C-Atomen verschlossen sind, Formamid, durch 1 oder 2 Alkylreste mit 1 bis 4 C-Atomen am Amidstickstoff substituierte aliphatische Carbonsäureamide mit 1 bis 10 C-Atomen, wie Dimethylformamid, Diethyl- oder Dibutylformamid, Benzoesäureamide, gegebenenfalls am Stickstoff durch einen Alkylrest mit 1 bis 4 C-Atomen substituierte cyclische 5- bis 7-gliedrige Amide, wie N-Methylpyrrolidon-2, N-Methylpiperidon-2, ε-Caprolactam, Triazinderivate, wie Melamin, Benzoguanamin, Acetoguanamin, Trialkylamine mit 1 bis 6 C-Atomen im Alkyl, N,N-$C_1$-$C_4$-Dialkyl-cyclohexylamine, Pyridin, Triphenylphosphin, Amidosulfonsäure, Imidazol oder Bortrifluorid. Gegebenenfalls können auch Mischungen dieser Komplexbildner verwendet werden.

Davon sind die N,N-disubstituierten Formamide mit 1 bis 4 C-Atomen im Alkyl, insbesondere das Dimethylformamid, und das 1,4-Dioxan besonders bevorzugt.

Für R kommen als Alkylreste mit 5 bis 11 C-Atomen beispielsweise Pentyl, Heptyl, 2-Ethylpentyl, Octyl, verzweigte Octylreste, Undecyl in Betracht.

Von den Resten für R sind besonders bevorzugt n-Heptyl, n-Octyl und 3,5,5-Trimethylpentyl.

Bei der Herstellung können anstelle der reinen Säurechloride zweckmäßig die technisch erhältlichen Gemische, die in der Regel mindestens 95% definiertes Säurechlorid Cl—COR enthalten, eingesetzt werden. Dabei wird das 3,5,5-Trimethylhexansäurechlorid im technischen Sprachgebrauch häufig als Isonansäurechlorid bezeichnet.

Im übrigen steht in der Formel I der Rest—O—OR aufgrund der entstehenden Reaktionsgemische bevorzugt in der p-Stellung mit einem Anteil an o-Verbindung.

Bei der Umsetzung des zu sulfonierenden Phenols mit $SO_3$ oder Chlorsulfonsäure wird vorteilhaft ein Molverhältnis von etwa 1 : 1 eingehalten, um beispielsweise Sulfonbildung und Disulfonierungen und weitere Nebenprodukte zu vermeiden. Die molare Menge an Sulfonierungsmittel sollte zweckmäßig um nicht mehr als 5 Mol% überschritten werden.

Der bei Verwendung von Chlorsulfonsäure entstehende Chlorwasserstoff kann ohne Schwierigkeiten durch Ausgasen, z. B. unter vermindertem Druck bei 10 bis 40 mbar, praktisch vollständig entfernt werden.

Die erhaltene Phenolsulfonsäure wird vorteilhaft direkt mit einem entsprechenden Säurechlorid bei 35 bis 45 °C, in molarem Verhältnis, berechnet auf eingesetztes Phenol, umgesetz. Nach der Beendigung der Reaktion wird, wie oben angegeben, der entstandene Chlorwasserstoff entfernt.

Die hier beschriebenen Sulfonierungen einschließlich der nachfolgenden Veresterungen können diskontinuierlich oder kontinuierlich durchgeführt werden. Bei der kontinuierlichen Ausführungsform kann man beispielsweise so vorgehen, daß man die Reaktionskomponenten in einem Rohrreaktor oder in einer Rührkesselkaskade zusammenführt.

Aus praktischen Gründen und wegen der Zersetzlichkeit der gewonnenen Acyloxybenzolsulfonsäuren, die als aktivierte Phenolester sehr hydrolyseempfindlich sind, ist es vorteilhaft, die erhaltenen flüssigen Acyloxybenzolsulfonsäuren in ihre Alkali- oder Erdalkalisalze zu überführen. Davon ist das Natriumsalz besonders bevorzugt.

Das erhaltene Salz kann ggf. in an sich üblicher Weise aus der wäßrigen Lösung in fester Form isoliert, werden.

Das Neutralisationsverfahren, bei dem Acyloxybenzolsulfonsäuren ohne nennenswerte Verseifung neutralisiert werden können, ist übrigens Gegenstand der nicht vorveröffentlichten EP-A-140251. Im einzelnen geht man so vor, daß man in Wasser gleichzeitig die flüssige Acyloxybenzolsulfonsäure und eine 5 bis 50 gew.%ige wäßrige Lösung von Alkali- oder Erdalkali in Form des Hydroxids, Carbonats oder Bicarbonats bei Temperaturen von 0 bis 60 °C, bevorzugt 10 bis 40 °C, unter Rühren so einlaufen läßt, daß ein pH-Wert-Bereich von 2,5 bis 7,0, bevorzugt 3,0 bis 5,5, eingehalten wird.

Diese Neutralisation kann diskontinuierlich oder kontinuierlich durchgeführt werden. Bei der kontinuierlichen Arbeitsweise werden die Komponenten Wasser, Acyloxybenzolsulfonsäure und Alkali in einem statischen oder dynamischen Mischer zusammengeführt.

Durch dieses Neutralisationsverfahren können stabile wäßrige Lösungen der Acyloxybenzolsulfonate in Konzentrationen von 20 bis 60 Gew.% hergestellt werden. Aus diesen Lösungen können die reinen Salze in

an sich üblicher Weise, beispielsweise durch Eindampfen, Walzentrocknen, Sprühtrocknen, Gefriertrocknen oder Wirbelbetttrocknen isoliert werden.

In einer besonders bevorzugten Ausführungsform wird diese spezielle Neutralisation in Gegenwart von 1 bis 2 Gew.%, bezogen auf die Acyloxybenzolsulfonsäure, von einem wasserlöslichen Phosphat, Phosphit, Tartrat oder einem Komplexbildner für Schwermetalle oder einem Polymeren aus Acrylsäure und/oder Maleinsäure durchgeführt. Dabei werden in der Regel die wasserlöslichen Natriumsalze verwendet.

Die wäßrigen Lösungen sind dann wesentlich weniger gefärbt und neigen bei der Weiterverarbeitung weniger zu Verfärbungen, wenn man die Neutralisation in Gegenwart dieser Stoffe durchführt. Im einzelnen seien beispielsweise genannt: Natriumdihydrogenphosphat, Dinatriumtartrat, Natriumhydrogentartrat, Natriumphosphit, unterphosphorige Säure, Nitrilotriessigsäure, Ethylendiamintetraessigsäure, Diethylentriaminpentaessigsäure, Hydroxyethylethylendiamintriessigsäure, Nitrilotrimethylenphosphonsäure oder Polycarbonsäuren aus Acrylsäure und/oder Maleinsäure und ihre Natriumsalze. Die verwendeten Polyacrylsäuren weisen K-Werte von 15 bis 120 und die Acrylsäure-Maleinsäure-Copolymerisate K-Werte von 30 bis 100 auf, jeweils gemessen als vollständig neutralisiertes Na-Salz in 1 gew.%iger wäßriger Lösung bei 25 °C.

## Beispiele

Die vorliegenden Beispiele werden mit technischem 3,5,5-Trimethylhexansäurechlorid (Isononanoylchlorid) durchgeführt. Andere Acylreste verhalten sich völlig analog. Der Gehalt der Reaktionsmischung an Acyloxybenzolsulfonsäure wird zweckmäßigerweise nach der Neutralisation mit wäßriger Natronlauge und Sprühtrocknung an den isolierten Natriumsalzen durch Zweiphasentitration nach DIN/ISO 2271 bestimmt Teile sind Gewichtsteile.

1. Allgemeine Vorschrift für Beispiele 1 bis 8 und Vergleichsbeispiele A und B :

94 Teile Phenol werden geschmolzen und mit Komplexbildner gemäß Tabelle 1 versetzt. Bei 45 bis 50 °C werden innerhalb von 1 bis 2 Stunden unter Kühlung 122 Teile Chlorsulfonsäure oder 84 Teile Schwefeltrioxid zugegeben. Es wird 1 Stunde lang bei 50 °C nachgerührt und dann werden innerhalb von 1 bis 2 Stunden bei maximal 45°C 176,5 Teile 3,5,5-Trimethylhexansäurechlorid zugegeben. Nach 1 Stunde wird unter vermindertem Druck bei 10 bis 20 mbar der gelöste Chlorwasserstoff praktisch vollständig entfernt.

100 Teile der erhaltenen rohen Acyloxybenzolsulfonsäure läßt man unter gutem Rühren zu 100 Teilen Wasser zulaufen. Gleichzeitig wird 50 gew.%ige wäßrige Natronlauge so zugetropft, daß sich in der wäßrigen Lösung ein pH-Wert von 3,0 bis 5,5 (Verfolgung mittels Glaselektrode) einstellt. Die Temperatur der Reaktionsmischung wird durch Kühlung unter 50°C gehalten. Nach dem Ende der Zugabe der Acyloxybenzolsulfonsäure wird die Lösung auf einen pH-Wert von 5,5 eingestellt. Das Natriumsalz wird durch Sprühtrocknung aus der wäßrigen Lösung isoliert.

## Beispiel C

In 73 Teilen Dimethylformamid werden 94 Teile Phenol gelöst und anschließend auf 40 °C erwärmt. Diese Mischung wird bei 40 bis 50 °C innerhalb zwei Stunden mit 119 Teilen Chlorsulfonsäure sulfoniert, 30 Minuten bei 50 °C nachgerührt und anschließend bei 50 °C im Vakuum entgast. Bei 40 bis 50 °C werden 176,5 Teile 3,5,5-Trimethylhexansäurechlorid zugefügt und nach einer Stunde der gelöste Chlorwasserstoff unter vermindertem Druck bei 10 bis 20 mbar und 50 °C praktisch vollständig entfernt.

Die rohe Acyloxybenzolsulfonsäure wird wie vorstehend beschrieben neutralisiert und das Natriumsalz durch Sprühtrocknen aus der wäßrigen Lösung isoliert.

Die Ergebnisse sind in Tabelle 1 zusammengefaßt. Die Ergebnisse zeigen, daß für eine großtechnische Verfahrensweise gegenüber den Vergleichsversuchen die Ausbeuten in nicht vorhersehbarer Weise verbessert wurden.

Tabelle 1

| Beisp. | $ClSO_3H$ | $SO_3$ | Komplexbildner | Tle. | = Mol%[2)] | Gehalt an Na-Salz[1)] in % |
|---|---|---|---|---|---|---|
| 1 | + | - | Dimethylformamid | 0,94 | 1,2 | 84,4 |
| 2 | + | - | Dimethylformamid | 1,88 | 2,5 | 86,2 |
| 3 | + | - | Dimethylformamid | 4,70 | 6,1 | 86,6 |
| 4 | - | + | Dimethylformamid | 0,94 | 1,2 | 83,2 |
| 5 | - | + | Dimethylformamid | 1,88 | 2,5 | 82,9 |
| 6 | - | + | Dioxan | 0,94 | 1,0 | 81,8 |
| 7 | + | - | Dioxan | 2,82 | 3,0 | 86,8 |

Tabelle (Fortsetzung)

| Beisp. | $ClSO_3H$ | $SO_3$ | Komplexbildner | Tle. | = Mol%[2)] | Gehalt an Na-Salz[1)] in % |
|---|---|---|---|---|---|---|
| 8 | - | + | Tetramethylharnstoff | 0,94 | 0,8 | 85,7 |
| Vergleichs-beispiele | | | | | | |
| A | + | - | - | - | - | 79,6 |
| B | - | + | - | - | - | 76,1 |
| C | + | - | Dimethylformamid | 73 | 100 | 79,4 |

1) Zweiphasentitration nach DIN/ISO 2271
2) bezogen auf $ClSO_3H$ oder $SO_3$

## Patentansprüche

1. Verfahren zur Herstellung der Alkali- und Erdalkalisalze von Acyloxybenzolsulfonsäuren der Formel I

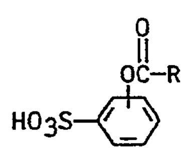

in der R einen geradkettigen oder verzweigten gesättigten Alkylrest mit 5 bis 11 C-Atomen oder einen Phenylrest bedeutet, durch Sulfonierung von Phenol mit $SO_3$ oder Chlorsulfonsäure, anschließende Veresterung und Salzbildung dadurch gekennzeichnet, daß man die Sulfonierung in Gegenwart von 0,2 bis 20 Mol% eines Komplexbildners für $SO_3$ oder Chlorsulfonsäure, bezogen auf das $SO_3$ oder die Chlorsulfonsäure, bei Temperaturen von 20 bis 80°C durchführt wobei man den Komplexbildner dem geschmolzenen Phenol zusetzt und anschließend das Sulfonierungsmittel zusetzt und die erhaltene Phenolsulfonsäure direkt mit einem Säurechlorid der Formel Cl-COR, inder R die für die Formel I gennanten Bedeutungen hat, bei 25 bis 55°C umsetzt und anschließend zur Neutralisation die erhaltene flüssige Acyloxibenzolsulfonsäure der Formel I mit einer wäßrigen Lösung von Alkali- oder Erdalkali in Form des Hydroxids, Carbonats oder Bicarbonats in Wasser bei Temperaturen von 0 bis 60°C unter guter Durchmischung so zusammenbringt, daß ein pH-Wertbereich von 3,0 bis 5,5 eingehalten wird, und man gegebenenfalls das erhaltene Salz in an sich üblicher Weise aus der wäßrigen Lösung isoliert.

2. Verfahren nach Anspruch 1, dadurch gekennzeichnet, daß man die Sulfonierung in Gegenwart von 0,6 bis 10 Mol% eines Komplexbildners für $SO_3$ oder Chlorsulfonsäure, bezogen auf das $SO_3$ oder die Chlorsul-

fonsäure, bei Temperaturen von 30 bis 60°C durchführt und die erhaltene Phenolsulfonsäure direkt mit einem Säurechlorid der Formel Cl-COR, in der R die für Formel I genannten Bedeutungen hat, bei 35 bis 45°C umsetzt und anschliessend die erhaltene Acyloxybenzolsulfonsäure zum Alkali- oder Erdalkalisalz neutralisiert.

3. Verfahren nach Anspruch 1, dadurch gekennzeichnet, daß man als Komplexbildner für das $SO_3$ oder die Chlorsulfonsäure ein N,N-disubstituiertes Formamid mit 1 bis 4 C-Atomen im Alkyl oder Dioxan zusetzt.

4. Verfahren nach Anspruch 1, dadurch gekennzeichnet, daß die Neutralisation bei Temperaturen von 10 bis 40°C durchgeführt wird.

5. Verfahren nach Anspruch 1 und/oder 4, dadurch gekennzeichnet, daß die Neutralisation in Gegenwart von 1 bis 2 Gew.%, bezogen auf die Acyloxybenzolsulfonsäure, eines löslichen Phosphats, Phosphits, Tartrats, eines Komplexbildners für Schwermetallsalze oder eines Polymers aus Acrylsäure und/oder Maleinsäure durchgeführt wird.

## Claims

1. A process for the preparation of an alkali metal or alkaline earth metal salt of an acyloxybenzenesulfonic acid of the formula I

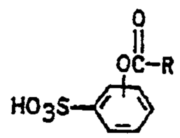

where R is straight-chain or branched, saturated alkyl of 5 to 11 carbon atoms or phenyl, by sulfonation of phenol with $SO_3$ or chlorosulfonic acid followed by esterification and salt formation, wherein the sulfonation is carried out in the presence of from 0.2 to 20 mol %, based on the $SO_3$ or the chlorosulfonic acid, of a complexing agent for $SO_3$ or chlorosulfonic acid, at from 20 to 80°C, the complexing agent being added to the molten phenol, followed by the sulfonating agent, and the resulting phenolsulfonic acid is reacted directly with an acyl chloride of the formula Cl-COR, where R has the meanings stated for the formula I, at from 25 to 55°C, and then in order to effect neutralization, the liquid acyloxybenzenesulfonic acid obtained, of the formula I, is combined with an aqueous solution of an alkali metal or alkaline earth metal hydroxide, carbonate or bicarbonate in water at from 0 to 60°C, with thorough mixing, in such a way that a pH of from 3.0 to 5.5 is maintained, and, if required, the resulting salt is isolated from the aqueous solution in a conventional manner.

2. A process as claimed in claim 1, wherein the sulfonation is carried out in the presence of from 0.6 to 10 mol %, based on the $SO_3$ or the chlorosulfonic acid, of a complexing agent for $SO_3$ or chlorosulfonic acid, at from 30 to 60°C, and the resulting phenolsulfonic acid is reacted directly with an acyl chloride of the formula Cl-COR, where R has the meanings stated for formula I, at from 35 to 45°C, and the acyloxybenzenesulfonic acid obtained is then neutralized to give the alkali metal or alkaline earth metal salt.

3. A process as claimed in claim 1, wherein an N,N-disubstituted formamide where alkyl is of 1 to 4 carbon atoms or dioxane is added as the complexing agent for the $SO_3$ or the chlorosulfonic acid.

4. A process as claimed in claim 1, wherein the neutralization is carried out at from 10 to 40°C.

5. A process as claimed in claim 1 or 4 or claims 1 and 4, wherein the neutralization is carried out in the presence of from 1 to 2% by weight, based on the acyloxybenzenesulfonic acid, of a soluble phosphate, phosphite or tartrate, a complexing agent for heavy metal salts or a polymer of acrylic acid and/or maleic acid.

## Revendications

1. Procédé de préparation des sels de métaux alcalins et de métaux alcalino-terreux d'acides acyloxybenzènesulfoniques de la formule I qui suit :

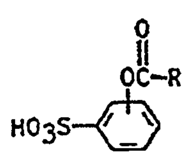

dans laquelle R représente un radical alkyle saturé, à chaîne droite ou à chaîne ramifiée, comportant de 5 à 11 atomes de carbone, ou un radical phényle, par sulfonation du phénol avec le $SO_3$ ou l'acide chlorosulfonique, estérification subséquente et salification, caractérisé en ce que l'on entreprend la sulfonation à des températures de 27 à 80°C, en présence de 0,2 à 20% molaires d'un agent complexant pour le $SO_3$ ou l'acide chlorosulfonique, par rapport au $SO_3$ ou à l'acide chlorosulfonique, opération au cours de laquelle on ajoute l'agent complexant au phénol fondu et on ajoute ensuite l'agent de sulfonation et on fait réagir directement l'acide phénolsulfonique obtenu sur un chlorure d'acide de la formule Cl-COR dans laquelle R possède les significations qui lui ont été attribuées à propos de la formule I, à 25-55°C et on réunit ensuite, en vue de la neutralisation, l'acide acyloxybenzène sulfonique liquide obtenu de la formule I avec une solution d'un métal alcalin ou d'un métal alcalino-terreux, sous forme de l'hydroxyde, du carbonate ou du bicarbonate, dans l'eau, à des températures de 0 à 60°C, sous bonne agitation, en manière telle que l'on maintienne le pH dans la plage des valeurs de 3,0 à 5,5 et l'on isole éventuellement le sel obtenu de la solution aqueuse d'une manière en soi usuelle.

2. Procédé suivant la revendication 1, caractérisé en ce que l'on entreprend la sulfonation à des températures de 30 à 60°C, en présence de 0,6 à 10% molaires d'un agent complexant pour le $SO_3$ ou l'acide chlorosulfonique, par rapport au $SO_3$ ou à l'acide chlorosulfonique et on fait réagir directement l'acide phénolsulfonique obtenu sur un chlorure d'acide de la formule Cl-COR dans laquelle R possède les significations qui lui ont été précédemment attribuées à propos de la définition de la formule I, à 35-45°C et on neutralise ensuite l'acide acyloxybenzène sulfonique obtenu en sel de métal alcalin ou de métal alcalino-terreux.

3. Procédé suivant la revendication 1, caractérisé en ce que l'on ajoute un formamide N,N-disubstitué avec un radical alkyle, à 1-4 atomes de carbone, ou le dioxanne, à titre d'agent complexant pour le $SO_3$ ou l'acide chlorosulfonique.

4. Procédé suivant la revendication 1, caractérisé en ce que l'on entreprend la neutralisation à des températures de 10 à 40°C.

5. Procédé suivant les revendications 1 et/ou 4, caractérisé en ce que l'on entreprend la neutralisation en présence de 1 à 2% en poids, par rapport à l'acide acyloxybenzène sulfonique, d'un polymère de l'acide acrylique et/ou de l'acide maléique, d'un agent complexant pour les sels de métaux lourds, d'un tartrate, d'un phosphite ou d'un phosphate soluble.